# EUROPEAN PATENT SPECIFICATION

(11) **EP 2 179 732 B1**
(45) Date of publication and mention of the grant of the patent: **14.05.2014**
(21) Application number: 08778967.3
(22) Date of filing: 16.06.2008
(51) Int. Cl.: A61K 31/365, A61K 31/40, A61K 45/06, A61P 3/06

(54) **PHARMACEUTICAL COMPOSITION COMBINING AN ENZYME HMG-COA REDUCTASE INHIBITING AGENT AND A GASTROINTESTINAL LIPASE ENZYME INHIBITING AGENT**
PHARMAZEUTISCHE ZUSAMMENSETZUNG MIT KOMBINATION AUS EINEM HMG-COA-REDUKTASEHEMMER UND EINEM GASTROINTESTINALEN LIPASEHEMMER
COMPOSITION PHARMACEUTIQUE COMPRENANT UN AGENT INHIBITEUR DE L'ENZYME HMG-COA REDUCTASE ET UN AGENT INHIBITEUR DE L'ENZYME LIPASE GATROINTESTINAL

(30) Priority: 15.06.2007 MX MX07007283
(43) Date of publication of application: 28.04.2010
(73) Proprietor: PPTM INTERNATIONAL S.à r.l., 1253 Luxembourg (LU)
(72) Inventor: GARCÍA ARMENTA, María Elena, C.P. 45020 Guadalajara, Jalisco (MX); SANTOS MURILLO, Josefina, C.P. 44600 Zapopan, Jalisco (MX); ALVAREZ OCHOA, Víctor Guillermo, C.P. 45222 Zapopan, Jalisco (MX)
(74) Representative: Carvajal y Urquijo, Isabel
(86) International application number: PCT/MX2008/000077
(87) International publication number: WO 2008/153374

(56) References cited:
- WO-A1-2008/039051
- WO-A2-2006/109170
- US-A1- 2006 148 721
- ZHI J ET AL: "Pharmacokinetic evaluation of the possible interaction between selected concomitant medications and orlistat at steady state in healthy subjects" JOURNAL OF CLINICAL PHARMACOLOGY, LIPPINCOTT CO, HAGERSTOWN, MD, US, vol. 42, no. 9, 1 September 2002 (2002-09-01), pages 1011-1019, XP002294531 ISSN: 0091-2700
- WIERZBICKI ANTHONY S ET AL: "Usefulness of Orlistat in the treatment of severe hypertriglyceridemia" AMERICAN JOURNAL OF CARDIOLOGY, CAHNERS PUBLISHING CO., NEWTON, MA, US, vol. 89, no. 2, 15 January 2002 (2002-01-15), pages 229-231, XP009136322 ISSN: 0002-9149
- WIERZBICKI A.S. ET AL.: 'Usefulness of orlistat in the treatment of severe hypertriglyceridemia' THE AMERICAN JOURNAL OF CARDIOLOGY vol. 89, no. 15, 2002, pages 229 - 231, XP009136322
- ZHI J. ET AL.: 'Pharmacokinetic evaluation of the possible interaction between selected concomitant medications and orlistat at steady state in healthy subjects' J. CLIN. PHARMACOL. vol. 42, 2002, pages 1011 - 1019, XP002294531
- HATZITOLIOS A. ET AL.: 'Efficacy of omega-3 fatty acids, atorvastatin and orlistat in non-alcoholic fatty lisee disease with dyslipidemia' INDIAN J. GASTROENTEROL. vol. 23, 2004, pages 131 - 134, XP009136327

## Description

### FIELD OF INVENTION

The present invention is applied to pharmaceutical industry and discloses a pharmaceutical composition comprising a synergic combination of a HMG-CoA reductase enzyme inhibiting agent, namely Atorvastatin and a gastrointestinal lipase enzyme inhibiting agent, namely Orlistat, which are formulated in a single dosage unit, which is indicated for treatment and control of dyslipidemias (hypercholesterolemia and hypertriglyceridemia) and related diseases.

The combination of above mentioned active principles produces a larger synergic effect when they are administered together in a single dose unit unlike when they are independently administered, thus causing benefits such as: lower concentrations of administered active principles, higher action fastness and lower risks that side effects are manifested.

### BACKGROUND OF INVENTION

Obesity is a multifactorial ethiopathogeny syndrome characterized by an increase in fatty tissue. This abnormality in body composition is accompanied by several pathologic manifestations. It has been reported that obesity is clearly associated with arterial hypertension, hypercholesterolemia, hypertriglyceridemia, non-insulin dependent diabetes mellitus, an increase in certain cancers and other medical problems. Consequently, this pathology reduces life expectations in those suffering it directly through its associated diseases.

A number of studies have analyzed the impact of obesity intensity as a main cause of mortality. Most increases in morbility and mortality are produced in mild to severe obesity (IMC>30 kg/m²), that is, overweight higher than 20% of acceptable weight. In magnitude, a higher effect is observed in an increase of cardiovascular diseases that constitute the first cause of death in many countries as is well known.

**Mortality according to weight variations, being 100 for average weights.**

| Overweight: | Men: | Women: |
|---|---|---|
| 20% | 121 | 123 |
| 30% | 137 | 138 |
| 40% | 162 | 163 |
| 50% | 210 | |

**Increase in risk, according to obesity in men considering 100 for average weights**

| | 20% weight above average | 40% weight above average |
|---|---|---|
| Coronary disease | 128 | 175 |
| Cerebral vascular injury | 116 | 191 |
| Cancer | 105 | 124 |
| Diabetes mellitus | 210 | 300 |
| All causes | 121 | 162 |

It has been a cause of controversy to solve whether obesity by itself is a risk factor regardless of certain diseases, such as atherosclerotic coronary cardiopathy, or is exerting its influence as a conditioning element of other factors especially: arterial hypertension, diabetes mellitus and dyslipidemias.

The Framingham study prospectively demonstrated that for each 10% of weight increase, blood pressure increases 6.5 mm. Hg, plasma cholesterol 12 mg./dL. and glycemia 2 mg./dL.

The association between obesity and arterial hypertension is a frequent fact. There are longitudinal studies which demonstrate that weight increase produces a significant increase in blood pressure, while a weight decrease in obese patients reduced pressure figures.

Clinical and epidemiological experience has demonstrated an indisputable association between obesity and non-insulin dependent diabetes mellitus and glucose impairment. Mild degrees of obesity may increase the risk of suffering diabetes mellitus up to 10-fold and the risk grows as obesity intensity is higher. Obesity type is also related to body fat distribution, the risk being higher in thoracoabdominal type obesity.

Obesity is remarkably among the most frequent secondary dyslipidemias. This is associated with the insulin-resistance syndrome commonly observed with fatty tissue excess, moreover when a thoracoabdominal or visceral distribution is present.

More frequent observation is hypertriglyceridemia, with a slight increase in total cholesterol, but with a remarkable decrease in HDL cholesterol (high density lipoproteins) and therefore, an increase in total cholesterol / HDL cholesterol ratio. Triglyceride increase is due to a higher hepatic synthesis, coming from an increase in free fatty acid supply in a hyperinsulinemia state by insulin resistance. VLDL cholesterol secretion is increased (very low density lipoproteins) and therefore hypertriglyceridemia is remarkable. HDL cholesterol reduction is explained by hypertriglyceridemia, since in those circumstances and by a lipid intravascular transfer, HDL receive triglycerides and accelerate their catabolism through a higher activity of hepatic lipase enzyme. On the other hand, something similar happens with LDL cholesterol (low density lipoproteins), since LDL receive triglycerides, which are partially metabolized by hepatic lipase enzyme and they are transformed in small and dense LDL which have a higher atherogenic potential (higher susceptibility to oxidation and lower affinity with apolipoprotein B receptors).

Meta-analysis studies recently tend to demonstrate that high triglycerides constitute a risk in general population and moreover in diabetic subjects and women. Regardless whether triglycerides are or not a risk factor, its association with HDL deficit and small and dense LDL production with a demonstrated physiopathological correlation explain the risk increase in these patients.

Weight reduction in dyslipidemic obese subjects is associated with a notorious improvement in dyslipidemia, with triglyceride decrease and HDL cholesterol increase. If response is partial and moreover when there are other associated risk factors, a suitable pharmacologic therapy for existing dyslipidemia type shall be determined.

On the other hand, some studies report that there are risk populations which although treated with statins, only about one half reach the objective of decreasing LDL cholesterol. Most patients treated with statins present an increase in LDL cholesterol reduction power upon increasing the administered dosages; however, even when using the maximum recommended doses for more powerful statins, LDL cholesterol figures of many patients remain above the established objectives. Further, some patients present a low response phenomenon to statins, which is observed in a limited number of patients treated where exaggerated intestinal cholesterol absorption is possibly having any influence.

Therefore, in spite of its efficacy, the isolated inhibition of cholesterol synthesis with statins has limitations which prevent to achieve the pharmaceutical objectives in a significant percentage of high risk subjects. Therefore, a method for reduction of cholesterol which is complementary to synthesis inhibition is desirable. This is logically, the interference with cholesterol intestinal absorption. In fact, cholesterol homeostasis in organism is kept by equilibrating the steroid endogenous synthesis with intestinal absorption and with biliary secretion of biliary acids and cholesterol.

However, since biliary acids are efficiently reabsorbed and one part of the biliary cholesterol is also absorbed in gut, global cholesterol balance depends on inputs (synthesis and diet) which are equilibrated with losses (fecal removal). Obviously, the amount of excreted cholesterol in feces fully depends on intestinal absorption efficiency (biliary and diet cholesterol at the same time).

In any case under normal conditions, absorbed cholesterol mass in intestine (biliary and diet) is comparable with that synthesized in all the organism, therefore it may be stated that intestine and liver (organ where most part of cholesterol is synthesized) are two main sources of cholesterol of similar magnitude.

In the light of the above, regulation of cholesterol intestinal absorption is of increasing interest as a therapeutic objective to reduce cholesterol and triglyceride figures.

Cholesterol is an insoluble molecule, thus its intestinal absorption assumes certain complexity which involves emulsification, ester bond hydrolysis (when sterified) by intervention of hydrolase specific pancreatic enzyme, micellar solubilization, proximal jejune absorption, enterocyte cytoplasm reesterification and lymph transport through chylomicrons.

Apart from cholesterol in food (about 300 mg. daily in Western diet), intestinal cholesterol also proceeds from two endogenous sources: bile, which contributes with about 1,000 mg per day and intestinal epithelial desquamation, which provides about 300 mg more.

Another remarkable feature of cholesterol absorption is its relative inefficiency, which in average absorbs only 40%, although with a variability oscillating between 20 and 80%. In any case, absorbed cholesterol has liver as final destination, the main organ responsible of LDL cholesterol production and clearance, therefore being apparent that any variation in cholesterol absorption efficiency has a potential influence over circulating LDL cholesterol figures.

Most of the steps which intervene in cholesterol intestinal absorption are currently already characterized, but the limiting factor in its absorption is still unknown (the passage from lumen to enterocyte), and three phases may be distinguished: intraluminal, mucosa and intracellular.

Within intraluminal phase, sterified cholesterol is as insoluble as triglycerides, being present as oil but the main lipolytic product of its hydrolysis by intervention of pancreatic carboxyl ester lipase enzyme is free cholesterol, which still has a very low solubility. This justifies a full dependency in cholesterol absorption in the solubilizing capacity of biliary acid micelles. Micelle formation by biliary acids and bile fosfolipids allows hydrophobic molecule transportation (cholesterol and other steroids, fatty acids, monoglycerides) in aqueous medium from intestinal content (in the same way than blood lipoproteins) and is a well-known interesting physical-chemical process since several decades ago.

Micellar solubilization is indispensable for cholesterol to be diffused through a slight mucous barrier which covers the intestinal microvilli surface. Micelles are disaggregated in this zone, therefore cholesterol monomers are available to be captured by enterocyte.

Interference with cholesterol micellar solubilization reduces its bioavailability and is one of the therapeutic embodiments to inhibit its intestinal absorption.

The second phase of absorption process, mucous phase, comprises a step from cholesterol molecules from lumen to enterocyte cytoplasm through brush border. Traditionally, it has been believed that this would take place because of a passive diffusion process, although some studies would suggest the existence of a proteinaceous transporter.

Arguments in favor of an active transportation protein at least were based in cholesterol absorption specificity since other quite structurally similar sterols being present in diet, phytosterols and marine sterols are very less absorbed.

Phytosterols, such as β-sitosterol, campesterol and stigmasterol, are found in many vegetable products and represent up to 50% of total sterol daily intake; β-sitoesterol absorption is known from long time ago that differs from cholesterol only for an ethyl group addition in C:24, being lower than 5%.

Although phytosterols are absorbed in a much more inefficient way than cholesterol, absorption mechanism is similar and, since its origin is exclusively dietary, phytosterol / cholesterol quotient in plasma may be used as a reliable marker for cholesterol absorption.

Seafood marine sterols are also less efficiently absorbed than cholesterol. It is known currently that this selectivity is mediated through two membrane transporters, ABCG5 y ABCG8, which jointly act as an heterodimer, "pumping" sterols out of hepatocytes (contributing to cholesterol biliary secretion) and from enterocytes (returning absorbed sterols to intestinal lumen).

Therefore, an intestinal "return" mechanism from absorbed sterols to lumen is known, but not the exact absorption mechanism although it is well assumed that it deals with a brush border active transporter.

Along intracelular phase, cholesterol which is captured by enterocytes and is not returned to lumen through ABCG5/8 path is diffused to endoplasmatic reticulum and then reesterified by acetyl CoA: cholesterol acyltransferase-2 (ACAT-2) enzyme, which fulfills the same function as hepatocytes.

Cholesterol intracelular traffic wherein an increasing number of transport, gene transcription regulating and activating proteins is intervening, controls its cellular metabolism in enterocytes in the same way than cells from other organs, in such a way that an increase thereof stimulates ACAT activity, inhibits steroid endogenous synthesis and downregulates LDL receptor expression.

Final step is reesterified cholesterol incorporation by ACAT, together with a free cholesterol low ratio, to nascent chylomicrons, in association with triglycerides and Apo-B and its secretion to lymph.

Chylomicron assembly is a complex physico-chemical process wherein microsomal triglyceride transfer protein (MTP) action is indispensable. Logically, both ACAT and MTP are therapeutic objectives to intend a reduction of cholesterol intestinal absorption.

Absorbed dietary and biliary cholesterol is eventually transported by chylomicron remainders to liver, where it exerts significant regulating effects over steroid homeostasis.

Essentially, the arrival of intestinal cholesterol growing amounts to liver inhibits cholesterol endogenous synthesis as the main compensatory effect, although it may also stimulate biliary acid production and cholesterol biliary excretion, in such a way that substantial variations in cholesterol intake induce limited fluctuations of LDL cholesterol serum concentrations.

In any case, cholesterol absorption regulation is of key importance in lipid metabolism since its fecal excretion (equivalent to an absorption failure) is the main path for cholesterol removal from organism.

Studies in patients with high or low cholesterol absorption rates suggest that those with hyperabsorption show a higher response to cholesterolemia than dietary cholesterol and a lower efficacy to statin treatment, due to a downregulation of cholesterol synthesis by a larger amount of intestinal origin cholesterol arriving to liver, thus having little available enzymatic activity to be inhibited by statins.

The knowledge of molecular mechanisms of this process allows a drug design in combination directed to inhibit cholesterol absorption.

Current treatment scheme assigned by physician to patients who suffer dyslipidemias is a change in life style, which includes diet and exercise. However, a pharmacological treatment is additionally of relevant importance which currently includes administering hypocholesterolemic drugs such as HMG-CoA enzyme inhibitors, as well as fibrates, which reduce triglyceride levels present in blood.

However, coadministering both drugs with concentrations and high dose amounts may cause severe side effects such as: hepatic alterations and others, such as rhabdomyolysis which may lead to death. Wierzbicki A.S. et al. 2002, discloses the usefulness of orlistat as add-on therapy in the treatment of severe hypertriglyceridemia.

### SUMMARY OF INVENTION

Development of the pharmaceutical composition below described was carried out in order to provide a pharmaceutical option which achieves an improvement in dyslipidemia indexes (hypercholesterolemia and hypertriglyceridemia), obesity, overweight and therefore, to achieve a better life quality in patients which suffer said pathologies, being further capable of reducing vascular complications and to decrease the risk of manifesting side effects.

### DETAILED DESCRIPTION OF INVENTION

It is well known that activity of HMG-CoA reductase enzyme inhibitors is specific for reducing cholesterol serum levels and has little or no activity over triglyceride level decrease; therefore it is advisable in current treatments a coadministration of HMG-CoA reductase enzyme inhibitors with fibrates, which have a proved therapeutic effect over triglycerides; however, by knowing the molecular mechanism of cholesterol the design of new combined drugs which exert a synergic efficient activity with lower risk of manifesting side effects was eased.

In the light of the above and in order to suppress all drawbacks present when administering these active principles (HMG-CoA reductase enzyme inhibitors and fibrates), the development of a pharmaceutical composition subject of present invention was carried out, which comprises a synergic combination of a HMG-CoA reductase enzyme inhibiting agent and a gastrointestinal lipase inhibiting agent; which produces a satisfactory therapeutic effect when administered together with an oral single dosage unit, generating benefits such as: lower concentrations of formulated active principles, lower administered dosages, higher action fastness, higher therapeutic effect efficiency, lower interaction risk and lower risk of manifesting side effects.

**HMG-CoA reductase enzyme inhibiting agents** (3-hydroxyl-3-methylglutaryl coenzyme A reductase) also known as *statins,* are transformed in the first choice drugs for many patients with dyslipidemias, because of their excellent tolerance and efficacy over lipid parameters.

Statins help to block HMG-CoA reductase enzyme activity, which makes organism to produce less cholesterol. When cholesterol production is delayed, the liver starts to produce more LDL receptors. These receptors capture LDL particles in blood, thus reducing LDL cholesterol amount in blood stream. LDL reduced levels ("bad cholesterol") may lead to lower triglyceride levels and higher HDL cholesterol levels ("good cholesterol").

There are studies which unequivocally demonstrate that cholesterol reduction in low density lipoproteins (LDL) reduces deadly or non-deadly myocardium infarctions.

**Atorvastatin** is a selective and competitive inhibitor of HMG-CoA reductase, the limiting enzyme responsible of converting 3-hydroxy-3-methyl-glutarylcoenzime A in mevalonate, an sterol prodrug, including cholesterol.

Triglycerides and cholesterol are incorporated into VLDL in the liver and released into plasma for peripheral tissue distribution. Low-density lipoproteins (LDL) are formed from VLDL.

Atorvastatin reduces cholesterol and lipoprotein plasma levels by inhibiting HMG-CoA reductase enzyme activity and cholesterol synthesis in liver and increasing in cell surface the number of hepatic receptors for LDL, which leads to an increase in absorption and LDL catabolism.

Atorvastatin reduces LDL production and the number of LDL particles, and produces a deep and sustained increase in LDL receptor activity, together with a beneficial modification in circulating LDL quality. The drug is efficient in LDL cholesterol reduction in patients with homozygotic familial hypercholesterolemia, a population which does not respond usually to hypolipemic medication.

In a study of dose-response, Atorvastatin has demonstrated to reduce total cholesterol (30-46%), LDL cholesterol (41-61%), apolipoprotein B (34-50%) and triglycerides (14-33%) and producing less variables in HDL cholesterol and apolipoprotein A. These results agree with those observed in patients with heterocygotic familial hypercholesterolemia, several forms of non-familial hypercholesterolemia and mixed hyperlipidemia, including patients with non-insulin dependent diabetes mellitus. Total cholesterol, LDL cholesterol and apolipoprotein B reductions have demonstrated to reduce cardiovascular event risk and cardiovascular mortality.

Atorvastatin is a selective and competitive inhibitor of hydroxymethylglutaryl-coenzyme A reductase (HMG-CoA reductase). This enzyme is responsible of HMG-CoA conversion to mevalonate, a sterol prodrug including cholesterol. HMG-CoA reductase inhibition reduces mevalonate amounts and consequently cholesterol hepatic levels. This has effect in circulation, the final consequence being cholesterol reduction associated with LDL.

Atorvastatin is rapidly absorbed after oral administration, maximum plasma concentrations are reached after 1 to 2 hours. Absorption degree is increased proportionally with Atorvastatin dosage. Absolute atorvastatin bioavailability is about 12% and systemic availability of HMG-CoA reductase enzyme inhibiting activity is about 30%. The low systemic availability is attributed to a presystemic clearance in gastrointestinal mucose and/or a first stage hepatic metabolism. It is metabolized by cytochrome P450 to their ortho and parahydroxylated derivatives and to different beta-oxidation products.

**Gastrointestinal lipase inhibiting agents** manifest their therapeutic activity by selectively limiting the digestive absorption of certain nutrients which, as fat, seem to result determinant in obesity and dyslipidemia development. More than 95% of the 50 to 120 grams of fat consumed by an adult in food in Western developed countries are formed by long-chain triglycerides.

All steps are required for fat absorption; however, digestive hydrolysis through lipase activity is the authentically critical issue, since triglycerides shall be transformed in two free fatty acid molecules and one of monoacylglycerol to be absorbed.

**Orlistat** is a potent, specific long-action inhibitor for gastrointestinal lipases. It exerts its therapeutic activity in stomach and small intestine lumen by forming a covalent bond with serine active site in gastric and pancreatic lipases. The absence of said enzymes inactivates fat hydrolysis contained in diet as triglycerides, thus preventing that these are transformed in absorbable free fatty acids and monoglycerides, thus causing that they are excreted without being digested.

Absorption degree is minimum, intact Orlistat concentrations have not been detectable (< 5 ng./mL.) at 8 hours after oral administration. Distribution volume has not been capable of determining since drug absorbed amount is minimal and has not a defined systemic pharmacokinetics. In vitro, Orlistat is attached in >99% to plasma proteins (lipoproteins and albumin are the main attachment proteins).

It is possible that Orlistat is mainly metabolized in gastrointestinal wall. From the minimal fraction of systemically absorbed dose, two main metabolites: M1 (lactone ring hydrolysation in position 4) and M3 (M1 with N-formyl leucine radical removed), represent about 42% of total plasma concentration.

This pancreatic lipase inhibitor which causes a useful fat misabsorption in obesity treatment reduces cholesterol absorption, assumedly because it is entrapped in an intraluminal oily phase which prevents its access to micelles. This effect probably contributes to cholesterolemia reduction which is observed after administration, which is commonly higher than predictable by caused weight loss.

HMG-CoA reductase enzyme inhibitor used in the pharmaceutical composition subject of present invention is active principle Atorvastatin, which is present in formulation in a concentration range from 1.0 mg. to 80.0 mg., being preferably used a concentration of about 10.0 mg. to 40.0 mg., per dose unit.

Gastrointestinal lipase inhibitor used in pharmaceutical composition subject of present invention is active principle Orlistat, which is present in formulation in a concentration range from 10.0 mg. to 360.0 mg., being preferably used a concentration of about 60.0 mg. to 120.0 mg., per dose unit.

The pharmaceutical composition protected by present invention is formulated to be orally administered in a single dosage unit in capsule or tablet form, wherein synergic combination of active principles: Atorvastatin and Orlistat in capsule or tablet form is comprised, as well as pharmaceutically acceptable excipients.

Said pharmaceutical composition has been developed in order to provide a pharmaceutical alternative for treatment and control of diseases such as: dyslipidemias (hypercholesterolemia and hypertriglyceridemia). The above described pharmaceutical composition provides significant advantages such as: lower concentrations of active principles contained in formulation, efficient control of cholesterol and triglyceride levels, lower risk of interactions, as well as a lower risk of side effects being manifest.

In order to assess the efficacy and tolerance of pharmaceutical composition subject of present invention, as well as the synergic effect of drug substances Atorvastatin and Orlistat, combined in a single dosage unit, a clinical comparative study was carried out wherein above mentioned active principles were separately administered, as well as a combination thereof.

### Comparative Study of Orlistat, Atorvastatin and a Combination of Orlistat / Atorvastatin in patients with Hypercholesterolemia and Hypertriglyceridemia.

This deals with a randomized follow-up one-year study which assessed the treatment effects with Orlistat, Atorvastatin and a combination of Orlistat/Atorvastatin in lipid profile, body weight and blood pressure in obese patients with hypercholesterolemia and hypertriglyceridemia.

**Methods.** Selected patients were obese with a Body Mass Index (IMC) >30 Kg/m²; with border (Total cholesterol 200-240 mg./dL.) or severe (Total cholesterol ≥ 240 mg./dL.) hypercholesterolemia; with hypertriglyceridemia (≥ 150 mg./dL.); normal pressure patients (systolic pressure <140 mm Hg and diastolic pressure < 90 mm Hg).

Patients were >50 years old. Patients were randomized for receiving in **Group 1:** Orlistat 1 capsule (120 mg.) in main meal. **Group 2:** received Atorvastatin 1 capsule (10 mg.) in main meal. **Group 3:** received a combination of Orlistat/Atorvastatin 1 capsule (120 mg./10 mg., respectively) in main meal.

Compliance with treatment was assessed by counting capsules and with follow-up visits every 3 months. Total cholesterol, LDL cholesterol, HDL Cholesterol, Triglycerides in serum and blood pressure during baseline visit were assessed, and at 6 months and 12 months of treatment. Waist index was assessed.

**Results.** 90 patients were enrolled (45 women and 45 men; with an average age of 57 years. Those 90 completed the study (45 women who were distributed in the following way, Group 1: 15, Group 2: 15, Group 3: 15 and 45 men who were distributed as follows, Group 1: 15, Group 2: 15, Group 3: 15), see Table 1.

**Table 1. Demographic characteristics of patients in study (N = 80).**

| | Group 1 (n=30) | | Group 2 (n=30) | | (Group 3 (n=30) | |
|---|---|---|---|---|---|---|
| | **W** | **M** | **W** | **M** | **W** | **M** |
| Feature | (n=15) | (n=15) | (n=15) | (n=15) | (n=15) | (n=15) |
| Age | 55(9) | 57(9) | 55(9) | 58(9) | 54(9) | 56(9) |
| Weight (Kg.) | 97(10) | 95(10) | 95(10) | 97(9) | 95(8) | 99(8) |
| Height (cm.) | 167(9) | 168(10) | 161(8) | 169(7) | 164(7) | 173(9) |
| Circumference (cm.) | 100(8) | 97(7) | 96(9) | 99(3) | 100(7) | 100(6) |

| | | | | | | |
|---|---|---|---|---|---|---|
| Group 1= Orlistat; Group 2= Atorvastatin; Group 3= Combination Orlistat/Atorvastatin. Values are expressed as average (SD). No significant differences were present among groups. | | | | | | |

Two patients from Group 1 showed adverse gastrointestinal events (fecal urgency); however there was no need to cancel the treatment. After two weeks of treatment the adverse gastrointestinal events disappeared.

Patients in Group 2 who received Atorvastatin did not show alterations as to serum transaminases or in creatinphosphokinase activity.

No adverse events were reported in Group 3.

No significant differences were present in demographic features, lipid profile, body weight, blood pressure within or among groups.

Results showed some significant variations within groups in lipid profile (Table 2) from baseline to 6 months of treatment in Total Cholesterol.

**Table 2. Comparison of lipid profile (cholesterol and triglycerides [mg./dL.]) baseline, 6 months and 1 year of treatment.**

| Component | Baseline | 6 months | 1 year | Baseline change at 1 year % |
|---|---|---|---|---|
| CT | | | | |
| Group 1 | 252 (25) | 232 (22) | 211 (25) | -13.0* |
| Group 2 | 265 (23) | 191 (21)* | 184 (23) | -27.0** |
| Group 3 | 268 (24) | 179 (23)** | 158 (29)** | -35.2*** |

| C-LDL | | | | |
|---|---|---|---|---|
| Group 1 | 188 (21) | 163 (21) | 152 (23) | -17.0* |
| Group 2 | 196 (25) | 151 (22)* | 104 (23) | -33.0** |
| Group 3 | 199 (22) | 119 (21)** | 100 (23)** | -45.2*** |

| C-HDL | | | | |
|---|---|---|---|---|
| Group 1 | 41 (3) | 44 (5) | 45 (6) | 4.8 |
| Group 2 | 42 (3) | 44 (5) | 46 (5) | 12.3** |
| Group 3 | 41 (3) | 48 (5)** | 55 (6) | 20.0*** |

| TG | | | | |
|---|---|---|---|---|
| Group 1 | 156 (36) | 120 (24) | 100 (22) | -30-3** |
| Group 2 | 160 (31) | 126 (29) | 125 (28) | -25.0* |
| Group 3 | 169 (28) | 111 (24)* | 80 (27) | -41.0** |

| | | | | |
|---|---|---|---|---|
| CT = Total Cholesteroll; Group 1= Orlistat, Group 2= Atorvastatin, Group 3= Orlistat / Atorvastatin; C-LDL = Low density lipoproteins; C-HDL = High density Lipoproteins; TG = Triglycerides. Values expressed as average (SD). * P 0.05 versus baseline ** P < 0.02 versus baseline. *** P < 0.01 versus baseline. | | | | |

Results also showed significant differences within baseline groups at 6 months of treatment: Body Mass Index, Waist Circumference, see Table 3.

**Table 3. Comparison of body weight (body mass index [IMC], waist circumference reduction [RCC] and weight loss [PP]) baseline, 6 months and 1 year of treatment.**

| | | | | Baseline changes (%) | |
|---|---|---|---|---|---|
| Measure | Baseline | 6 Months | 1 year | 6 Months | 1 Year |
| IMC (Kg./m²⁾ | | | | | |
| Group 1 | 33.5 (1.5) | 30.3 (1.4) | 27.3 (1.3) | -3.2* | -6.2** |
| Group 2 | 33.4 (1.5) | 31.3 (1.3) | 29.3 (1.4) | -2.3 | -4.1 * |
| Group 3 | 33.7 (1.5) | 30.0 (1.4) | 26.1 (0.8) | -3.7* | -7.6*** |
| | | | | | |

| RCC (cm.) | | | | | |
|---|---|---|---|---|---|
| Group 1 | --------- | -2.1 (0.7) | -4.1 (1.1) | -2.1 * | -4.6* |
| Group 2 | --------- | -1.5 (0.7) | -3.1 (1.1) | -1.5 | -3.7* |
| Group 3 | --------- | -3.9 (0.9) | -6.5 (4.9)*** | -3.9* | -6.1*** |
| | | | | | |

| PP | | | | | |
|---|---|---|---|---|---|
| Group 1 | --------- | -4.0 (1.2) | -8.6 (1.5) | -4.* | -4.6** |
| Group 2 | --------- | -3.7 (1.1) | -7.3 (1.2) | -3.7 | -4.6* |
| Group 3 | --------- | -8.5 (1.4)* | -13.5 (1.5)* | -8. *5 | -5.0*** |

| | | | | | |
|---|---|---|---|---|---|
| IMC = Body Mass Index; Group 1= Orlistat, Group 2= Atorvastatin, Group 3= Orlistat / Atorvastatin; RCC = Waist circumference reduction; PP = Weight loss. * P < 0.05 versus basaeline ** P < 0.02 versus baseline. *** P < 0.01 versus baseline. | | | | | |

Results showed significant changes in baseline blood pressure at 6 months and 1 year of treatment, see Table 4.

**Table 4. Blood pressure comparison (systolic [PAS] mm. Hg and diastolic [PAD] mm. Hg) baseline, 6 months and 1 year of treatment.**

| | | | | Baseline changes (%) | |
|---|---|---|---|---|---|
| Pressure | Baseline | 6 Months | 1 Year | 6 Months | 1 Year |
| Systolic | | | | | |
| Group 1 | 136 (4) | 130 (3) | 128 (3) | -6.0 | -16.0* |
| Group 2 | 133 (4) | 130 (4) | 127 (4) | -3.0 | -6.0* |
| Group 3 | 13 (5) | 123 (4) | 119 (4) | -13.0* | -17.0*** |
| | | | | | |

| Diastolic | | | | | |
|---|---|---|---|---|---|
| Group 1 | 90 (3) | 82 (3) | 82 (3) | -8.0 | -8.0* |
| Group 2 | 90 (4) | 85 (4) | 83 (3) | -5.0 | -7.0* |
| Group 3 | 90 (3) | 75 (4) | 71 (4)** | -15.0 | -19.0*** |

### Conclusions.

Orlistat is a gastrointestinal lipase inhibiting agent, indicated for long-term obesity management and its co-morbilities, producing a dose-dependent reduction in fatty diet absorption with a maximum inhibition of fatty absorption of 30% at a dosage of 120 mg once a day.

Atorvastatin is a HMG-CoA reductase inhibitor which produces a substantial reduction of C-LDL, together with a slight increase in C-HDL. It is generally well tolerated as monotherapy with a good safety and efficacy profile, with higher power than Simvastatin.

Obese patients with hypercholesterolemia and hypertriglyceridemia had benefit in this study by administering Orlistat and Atorvastatin alone and in combination with Orlistat / Atorvastatin. All parameters were significantly improved for the group which received a combination.

The 3 treatment groups improved significantly from baseline parameters. Treatment with a combination of Orlistat / Atorvastatin showed significantly higher reductions of Total Cholesterol, C-LDL, C-HDL, Triglycerides serum levels, Body Mass Index, Waist Circumference, Weight Loss. There was also a small but significant difference found in blood pressure. The use of combination allows a reduction of administered doses to patients, thus preventing the manifestation of secondary effects. On the other hand, the combination reduces the risk of cardiovascular events in obese patients with hypercholesterolemia. The combination shows an efficient synergistic effect therefore concentrations on each administered dose are lower than those when active principles are individually or independently administered.

## Claims

1. A pharmaceutical composition for use in the treatment and control of dyslipidemias, comprising the synergic combination of 1.0 mg to 80.0 mg of Atorvastatin, 10.0 to 360.0 mg of Orlistat and a pharmaceutically acceptable excipient, said composition being formulated in an oral single dosage unit.

2. The pharmaceutical composition for the use according to claim 1, **characterized in that** comprises from 10.0 to 40.0 mg of Atorvastatin.

3. The pharmaceutical composition for the use according to claim 1, **characterized in that** comprises from 60.0 mg to 120 mg of Orlistat.

4. The pharmaceutical composition for the use according to claim 1, **characterized in that** is found in a capsule or tablet pharmaceutical form.

5. The use of a pharmaceutical composition according to claim 1, for manufacturing a medicine for the treatment and control of dyslipidemias.

## Patentansprüche

1. Pharmazeutische Zusammensetzung zur Verwendung bei der Behandlung und Kontrolle von Dyslipidämien, umfassend die synergetische Kombination von 1,0 mg bis 80,0 mg Atorvastatin, 10,0 bis 360,0 mg Orlistat und einen pharmazeutisch verträglichen Hilfsstoff, wobei die genannte Zusammensetzung in einer Einzeldosiseinheit zur oralen Verabreichung formuliert ist.

2. Pharmazeutische Zusammensetzung zur Verwendung nach Anspruch 1, **dadurch gekennzeichnet, dass** sie 10,0 bis 40,0 mg Atorvastatin umfasst.

3. Pharmazeutische Zusammensetzung zur Verwendung nach Anspruch 1, **dadurch gekennzeichnet, dass** sie 60,0 mg bis 120 mg Orlistat umfasst.

4. Pharmazeutische Zusammensetzung zur Verwendung nach Anspruch 1, **dadurch gekennzeichnet, dass** sie in einer Kapsel- oder Tablettenarzneiform vorliegt.

5. Verwendung einer pharmazeutischen Zusammensetzung nach Anspruch 1 zur Herstellung eines Medikaments zur Behandlung und Kontrolle von Dyslipidämien.

## Revendications

1. Composition pharmaceutique pour le traitement et le contrôle des dyslipidémies, comprenant la combinaison synergique de 1,0 mg à 80,0 mg d'Atorvastatine, de 10,0 à 360,0 mg d'Orlistat et un excipient pharmaceutiquement acceptable, ladite composition étant formulée en une dose orale unique.

2. Composition pharmaceutique pour l'utilisation selon la revendication 1, **caractérisée en ce qu'**elle comprend de 10,0 à 40,0 mg d'Atorvastatine.

3. Composition pharmaceutique pour l'utilisation, selon la revendication 1, **caractérisée en ce qu'**elle comprend de 60,0 mg à 120 mg d'Orlistat.

4. Composition pharmaceutique pour son utilisation selon la revendication 1, **caractérisée en ce qu'**elle se trouve sous forme pharmaceutique de gélule ou comprimé.

5. Utilisation d'une composition pharmaceutique selon la revendication 1, pour la fabrication d'un médicament pour le traitement et contrôle des dyslipidémies.
